# EUROPEAN PATENT APPLICATION

(11) **EP 2 444 839 A1**
(43) Date of publication of application: **25.04.2012**
(21) Application number: 10789392.7
(22) Date of filing: 07.06.2010
(51) Int. Cl.: G02F 1/15, C08G 79/00, C07D 213/22

(54) **DISPLAY ELEMENT AND COLOR ELECTRONIC PAPER USING SAME**

(30) Priority: 18.06.2009 JP 2009145842
(71) Applicant: National Institute for Materials Science, Tsukuba-shi, Ibaraki 305-0047 (JP)
(72) Inventor: HIGUCHI, Masayoshi, Tsukuba-shi Ibaraki 305-0047 (JP); AKASAKA, Yumeno, Tsukuba-shi Ibaraki 305-0047 (JP)
(74) Representative: Calamita, Roberto
(86) International application number: PCT/JP2010/059638
(87) International publication number: WO 2010/147017

(57) **Abstract**

Provided are a display device which is advantageous not only in that it can smoothly switch display/non-display for a desired pattern and has a memory effect, but also in that the display device has a simple construction of the members thereof and can be driven at a low voltage, and exhibits high display contrast and can be increased in the display area, and a color electronic paper using the same.

A display device **characterized by** including, between a transparent substrate having a surface electrode and a substrate having a back electrode opposite to the surface electrode, a pattern display layer containing an organic-inorganic hybrid polymer represented by the following formula (I): 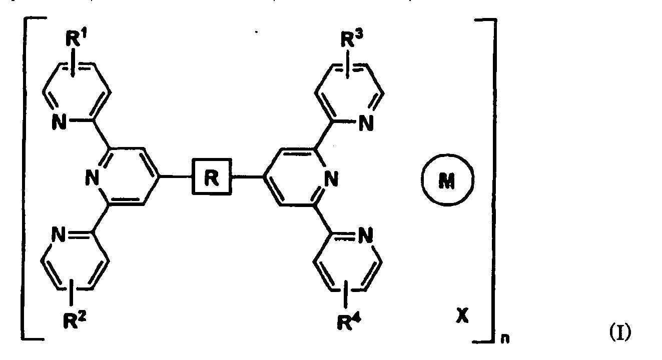 wherein M represents a metal ion, X represents a counter anion, R represents a spacer including a carbon atom and a hydrogen atom or a spacer for directly connecting two terpyridyl groups, each of R¹ to R⁴ independently represents a hydrogen atom or a substituent, and n represents an integer of 2 or more, which indicates a degree of polymerization, or the like, and a polymer gel electrolyte-containing layer.

## Description

### [Technical Field]

The present invention relates to a display device and a color electronic paper using the same.

In recent years, personal computers are being improved in the operating speed, network infrastructure is spreading, and data storage is being increased in the capacity and reduced in the cost, and therefore the information which has conventionally been provided through documents, images, and the like in the form of printed materials, such as paper, is available in the form of electronic information which is more convenient than the printed materials, and opportunities for reading electronic information are increasing.

As means for reading electronic information, conventional liquid crystal displays and CRTs (cathode ray tubes) and recently developed emission type displays, such as organic electroluminescence displays, are mainly used. Especially when the electronic information is document information, there is a need to gaze at the reading means for a relatively long period of time, and the means causing such behavior is not kind to humans. Generally, as drawbacks of the emission type display, there have been known problems in that flickering causes eyestrain, that it is inconvenient to carry the display, that the posture for reading is restricted to cause a need to concentrate the eyes on a static image, that long-time reading increases the power consumption, and the like.

As display means which can solve the problems, there has been known a reflective display having so-called memory properties such that an external light is utilized so as not to consume electric power for retaining the image for display, but the reflective display does not have satisfactory performance for the reasons mentioned below.

For example, a system using a polarizer, such as a reflective liquid crystal, has a reflectance as low as about 40% and has a problem about white display, and further many of the methods used for preparing the constituent members of this system are not simple. A polymer dispersed type liquid crystal requires a high voltage, and further utilizes a difference in refractive index between organic materials and hence cannot achieve a satisfactory contrast in the resultant image. Further, a polymer network type liquid crystal has problems in that a high voltage is required, that complicated TFT circuits are needed to improve the memory properties, and the like. Furthermore, a display device using an electrophoresis method requires a voltage as high as 10 V or more, and has a problem that the durability could become poor due to electrophoretic particle aggregation. As a method for performing color display using the above system, a method using a color filter has been known. This method is based on the principle of coloring using a color filter, and therefore cannot achieve bright white display.

Further, as a system which can achieve full color display and can be driven at a low voltage, an electrochromic system has been known. The electrochromic system can be driven at a voltage as low as 3 V or less, but, when full color display is attempted in this system, it is necessary to stack three layers of different colors, and therefore there is a fear that the complicated construction of the device increases the cost. A full color electrochromic device using plane mixing (see, for example, patent document 1) has been known, but this system cannot obtain a satisfactory contrast in the color display due to the plane mixing, and thus a method for performing multi-color display in a single layer is desired.

On the other hand, as an electrochromic device, a polymer material including a bis(terpyridine) derivative, a metal ion, and a counter anion has been proposed (see, for example, patent documents 2 to 4). For example, patent document 2 discloses that a bis(terpyridine) derivative having coordination properties and a metal ion together form a complex, so that an organic-inorganic hybrid polymer is formed, making it possible to control the color to be turned on or off.
[Patent document 1] JP-A-2003-270670
[Patent document 2] JP-A-2007-112957
[Patent document 3] International Publication No. WO2008/143324 pamphlet
[Patent document 4] International Publication No. W02008/081762 pamphlet

### [Disclosure of the Invention]

### [Problems that the Invention is to Solve]

Patent documents 2 to 4 suggest the applicability of the organic-inorganic hybrid polymer to a display device, but specific application of the polymer to a display device, such as a color electronic paper, has not yet been realized.

In view of the above, the present invention has been made, and a task is to provide a display device which is advantageous not only in that the display device can smoothly switch display/non-display for a desired pattern and has a memory effect, but also in that the display device has a simple construction of the members thereof and can be driven at a low voltage, and exhibits high display contrast and can be increased in the display area, and a color electronic paper using the same.

### [Means for Solving the Problems]

For solving the above problems, the present invention has the following characteristic features.

First: a display device characterized by including, between a transparent substrate having a surface electrode and a substrate having a back electrode opposite to the surface electrode, a pattern display layer containing an organic-inorganic hybrid polymer represented by the following formula (I) or (II):

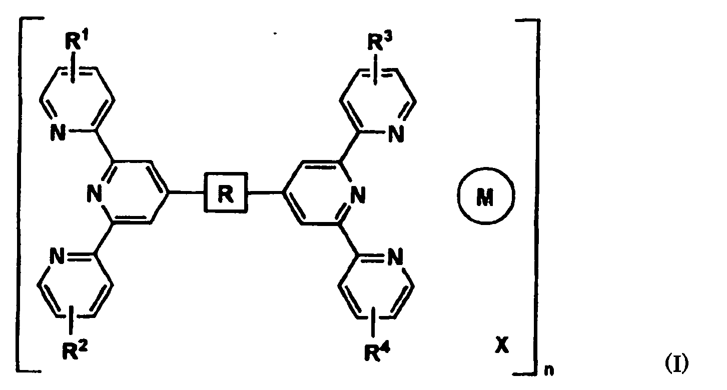

wherein M represents a metal ion, X represents a counter anion, R represents a spacer including a carbon atom and a hydrogen atom or a spacer for directly connecting two terpyridyl groups, each of R¹ to R⁴ independently represents a hydrogen atom or a substituent, and n represents an integer of 2 or more, which indicates a degree of polymerization,

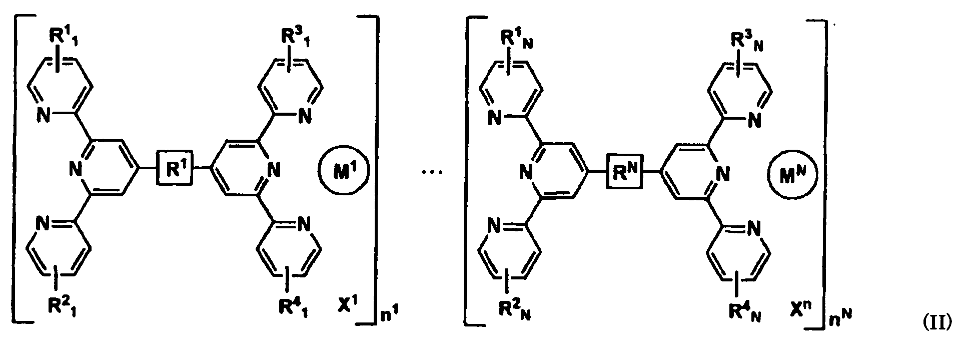

wherein each of M¹ to M^{N} (wherein N represents an integer of 2 or more) independently represents a metal ion, each of X¹ to X^{N} (wherein N represents an integer of 2 or more) independently represents a counter anion, each of R¹ to R^{N} (wherein N represents an integer of 2 or more) independently represents a spacer including a carbon atom and a hydrogen atom or a spacer for directly connecting two terpyridyl groups, each of R¹₁ to R¹_{N}, R²₁ to R²_{N}, R³_{N} to R³_{N}, and R⁴₁ to R⁴_{N} (wherein N represents an integer of 2 or more) independently represents a hydrogen atom or a substituent, and each of n¹ to n^{N} (wherein N represents an integer of 2 or more) independently represents an integer of 2 or more, which indicates a degree of polymerization, and a polymer gel electrolyte-containing layer.

Second: the above first display device, **characterized in that** the pattern display layer is formed on the surface of the transparent substrate having a surface electrode and/or the substrate having a back electrode.

Third: the above first or second display device, **characterized in that** the polymer gel electrolyte contains white particles.

Fourth: the above third display device, **characterized in that** the white particles are titanium dioxide fine particles.

Fifth: any one of the above first to fourth display devices, **characterized in that** the metal ion in the organic-inorganic hybrid polymer is at least one member selected from an iron ion, a cobalt ion, a nickel ion, a zinc ion, and a ruthenium ion.

Sixth: any one of the above first to fifth display devices, **characterized in that** the counter anion in the organic-inorganic hybrid polymer is at least one member selected from an acetate ion, a chloride ion, a hexafluorophosphate ion, a tetrafluoroborate ion, and a polyoxometalate.

Seventh: a color electronic paper characterized by including any one of the above first to sixth display devices.

Eighth: the above seventh color electronic paper, characterized by including a display portion having a size of 20 inches or more.

### [Advantage of the Invention]

In the invention, the display device using electrochromic properties of an organic-inorganic hybrid polymer (properties such that a substance changes in color due to electrochemical oxidation-reduction) can achieve pattern display, such as segment display or digital display, and has a simple construction of the members thereof and can be driven at a low voltage. In addition, the display device enables display to last for a long time after shutting the power source off (memory effect). Further, the addition of a white pigment, such as titanium dioxide fine particles, to the polymer gel electrolyte can enhance the display contrast. Moreover, a display having a large area, for example, a large-size color electronic paper having a side of 40 cm can be achieved.

### [Brief Description of Drawings]

[Fig. 1] Fig. 1 is a view diagrammatically showing an embodiment of the display device of the invention.
[Fig. 2] Fig. 2 is a photograph of the display devices obtained in Example 1 (right) and Example 2 (left).
[Fig. 3] Fig. 3 is a photograph of the display device obtained in Example 4.

### [Description of Reference Numerals]

1: Transparent substrate
2: Pattern display layer
3: Polymer gel electrolyte
4: Substrate

### [Best Mode for Carrying Out the Invention]

Hereinbelow, the present invention will be described in detail.

The organic-inorganic hybrid polymer of the formula (I) or (II) used in the invention includes a bis(terpyridine) derivative, a metal ion, and a counter anion.

The bis(terpyridine) derivative having coordination properties and the metal ion together form a complex, thus forming a state in which the bis(terpyridine) derivative and metal ion are alternately connected to each other (polymer complex).

The organic-inorganic hybrid polymer exhibits a color based on the charge-transfer absorption from the metal to the bis(terpyridine) derivative as a ligand. Specifically, when the organic-inorganic hybrid polymer is electrochemically oxidized, the color of the polymer disappears. On the other hand, when the organic-inorganic hybrid polymer in the colorless state is electrochemically reduced, the state of the polymer is turned to the colored state again. These phenomena can be repeatedly conducted.

R of the formula (I) and R¹ to R^{N} of the formula (II) are individually a spacer for connecting two terpyridyl groups, and by virtue of the spacer, the angle of the pyridyl group in the organic-inorganic hybrid polymer can be arbitrarily set, thus enabling material design for the organic-inorganic hybrid polymer.

With respect to the spacer, one having two terpyridyl groups directly connected thereto may be used, but a divalent organic group including a carbon atom and a hydrogen atom can be used, and examples of such divalent organic groups include aliphatic hydrocarbon groups, alicyclic hydrocarbon groups, aromatic hydrocarbon groups, and heterocyclic groups. Of these, preferred are arylene groups, such as a phenylene group and a biphenylene group. These hydrocarbon groups may have a substituent, e.g., an alkyl group, such as a methyl group, an ethyl group, or a hexyl group, an alkoxy group, such as a methoxy group or a butoxy group, or a halogen atom, such as chlorine or bromine. The spacer may further include an oxygen atom or a sulfur atom. The oxygen atom or sulfur atom has a modifying ability and hence is advantageous to the material design for the organic-inorganic hybrid polymer.

As preferred examples of the spacers, there can be mentioned divalent arylene groups represented by the following formulae (1) to (11).

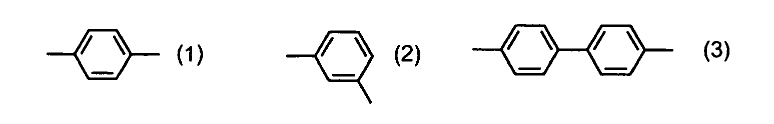

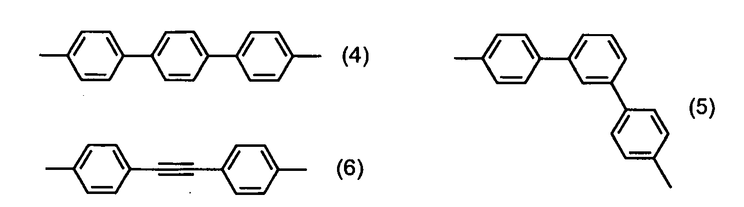

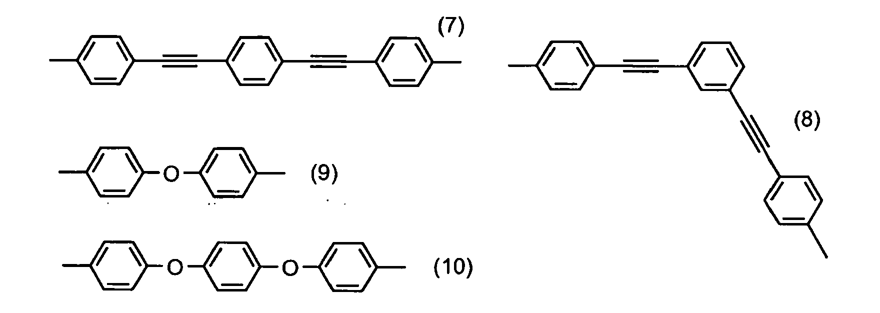

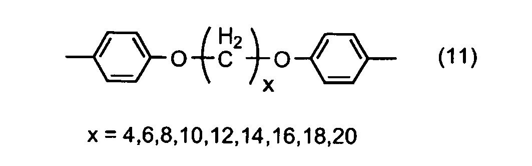

Examples of aliphatic hydrocarbon groups constituting the spacer include C₁-C₆ alkyl groups, specifically, a methyl group, an ethyl group, a n-propyl group, an i-propyl group, a n-butyl group, and a t-butyl group, and further, as the divalent organic group constituting the spacer, there can be used the above groups having a substituent, e.g., an alkyl group, such as a methyl group, an ethyl group, or a hexyl group, an alkoxy group, such as a methoxy group or a butoxy group, or a halogen atom, such as chlorine or bromine.

Examples of the metal ions M of the formula (I) and M¹ to M^{N} of the formula (II) include an iron ion, a cobalt ion, a nickel ion, a zinc ion, and a ruthenium ion. These metal ions not only can change in their valence due to a reduction reaction, but also individually have different oxidation-reduction potentials in the organic-inorganic hybrid polymer represented by the formula (I) above.

Examples of the counter anions X of the formula (I) and X¹ to X^{N} of the formula (II) include an acetate ion, a chloride ion, a hexafluorophosphate ion, a tetrafluoroborate ion, and a polyoxometalate. A counter anion makes up for the charge of the metal ion to render the organic-inorganic hybrid polymer electrically neutral.

Each of R¹ to R⁴ of the formula (I) and each of R¹₁ to R¹_{N}, R²₁ to R²_{N}, R³_{N} to R³_{N}, and R⁴₁ to R⁴_{N} of the formula (II) independently represents a hydrogen atom or a substituent, and examples of the substituents include a halogen atom, a hydrocarbon group, a hydroxyl group, an alkoxy group, a carbonyl group, a carboxylate group, an amino group, a substituted amino group, an amide group, a substituted amide group, a cyano group, and a nitro group. Examples of hydrocarbon groups include C₁-C₁₀ linear or branched alkyl groups, specifically, a methyl group, an ethyl group, a n-propyl group, an i-propyl group, a n-butyl group, and a t-butyl group, and further, as the substituent, there can be used the above hydrocarbon groups having a substituent, e.g., an alkyl group, such as a methyl group, an ethyl group, or a hexyl group, an alkoxy group, such as a methoxy group or a butoxy group, or a halogen atom, such as chlorine or bromine.

In the formula (I), n represents an integer of 2 or more, which indicates a degree of polymerization, and n is, for example, 2 to 5,000, preferably 10 to 1,000. In the formula (II), each of n¹ to n^{N} independently represents an integer of 2 or more, which indicates a degree of polymerization, and the sum of them, i.e., n¹ + n² + ... + n^{N} is, for example, 2 to 5,000, preferably 10 to 1,000.

The organic-inorganic hybrid polymer of the formula (I) or (II) can be produced by, for example, a method described in patent documents 2 to 4. For example, the organic-inorganic hybrid polymer of the formula (I) can be produced by a method in which a bisterpyridine derivative and a metal salt in acetic acid or methanol are heated at 150°C under reflux for about 24 hours. The reflux conditions vary depending on the selected spacer or metal salt, but the optimum conditions can be easily selected by those skilled in the art.

The organic-inorganic hybrid polymer synthesized by the above-mentioned method may be obtained in the form of powder by heating the mixture obtained by refluxing to evaporate the solvent. The powder has, for example, a color of violet and is in the reduction state. Such powder is easily dissolved in methanol and thus is easy to handle.

The organic-inorganic hybrid polymer of the formula (II) can be produced by, for example, a method including a step for heating the bisterpyridine derivatives respectively corresponding to the 1st to Nth of the formula (II) and the metal salts respectively corresponding to the 1 st to Nth individually in acetic acid and methanol under reflux, and a step for mixing together the 1 st to Nth (wherein N represents an integer of 2 or more) reaction mixtures obtained in the above step.

The polymer gel electrolyte used in the invention is a gel electrolyte using an organic solvent and a polymer. As the electrolyte used in the polymer gel electrolyte, preferred is a compound which is soluble in an organic solvent and which has a satisfactory electric conductivity (0.2 S/m or more), such as a lithium salt, a sodium salt, a potassium salt, or an ammonium salt, and examples of such compounds include lithium perchlorate, lithium tetrafluoroborate, lithium hexafluorophosphate, lithium trifluorosulfate, lithium hexafluoroarsenate, ammonium perchlorates, such as tetrabutylammonium perchlorate, tetraethylammonium perchlorate, and tetrapropylammonium perchlorate, and ammonium hexafluorophosphates, such as tetrabutylammonium hexafluorophosphate, tetraethylammonium hexafluorophosphate, and tetrapropylammonium hexafluorophosphate.

As the organic solvent, there can be used an organic solvent having a boiling point in the range of from 120 to 300°C such that, for example, after an electrolyte is formed, the organic solvent can remain in the electrolyte without suffering volatilization. Examples of such organic solvents include propylene carbonate, ethylene carbonate, ethylmethyl carbonate, diethyl carbonate, dimethyl carbonate, butylene carbonate, γ-butyrolactone, tetramethylurea, sulfolane, dimethyl sulfoxide, 1,3-dimethyl-2-imidazolidinone, 2-(N-methyl)-2-pyrrolidinone, hexamethylphosphoric triamide, N-methylpropionamide, N,N-dimethylacetamide, N-methylacetamide, N,N-dimethylformamide, N-methylformamide, butyronitrile, propionitrile, acetonitrile, acetylacetone, 4-methyl-2-pentanone, 2-butanol, 1-butanol, 2-propanol, 1-propanol, acetic anhydride, ethyl acetate, ethyl propionate, dimethoxyethane, diethoxyfuran, tetrahydrofuran, ethylene glycol, diethylene glycol, triethylene glycol monobutyl ether, tricresyl phosphate, 2-ethylhexyl phosphate, dioctyl phthalate, and dioctyl sebacate.

Of these, a cyclic carboxylate compound, such as propylene carbonate, ethylene carbonate, ethylmethyl carbonate, diethyl carbonate, dimethyl carbonate, butylene carbonate, or γ-butyrolactone, is preferably used.

As the polymer in which the electrolyte is dispersed, preferred is a polymer which is dissolved in or swells (gels) with the above-mentioned organic solvent added to the polymer and which has high transparency, and examples of the polymers include polymethacrylates, such as polymethyl methacrylate, polyethyl methacrylate, polybutyl methacrylate, polycyclohexyl methacrylate, and polyphenyl methacrylate, and polycarbonates.

In the invention, it is preferred that the electrolyte and the polymer are incorporated in an about 1:1 mass ratio into the polymer gel electrolyte.

In the invention, from the viewpoint of further increasing the display contrast to improve the visibility, it is preferred that the polymer gel electrolyte contains white particles. Examples of the white particles applicable to the invention include titanium dioxide (anatase type or rutile type), barium sulfate, calcium carbonate, aluminum oxide, zinc oxide, magnesium oxide, zinc hydroxide, magnesium hydroxide, magnesium phosphate, magnesium hydrogenphosphate, alkaline earth metal salts, talc, kaolin, zeolite, and acid clay.

In the invention, among the above-mentioned white particles, titanium dioxide, zinc oxide, or zinc hydroxide is preferably used. Further, there can be used titanium dioxide which has been subjected to surface treatment using an inorganic oxide {Al₂O₃, AIO(OH), SiO₂, or the like}, or titanium dioxide which has been subjected to the above surface treatment and further has been treated with an organic substance, such as trimethylolethane, triethanolamine acetate, or trimethylcyclosilane.

Of these white particles, from the viewpoint of prevention of the occurrence of discoloration at high temperatures and the reflectance of the device affected by the refractive index, titanium oxide or zinc oxide is more preferably used.

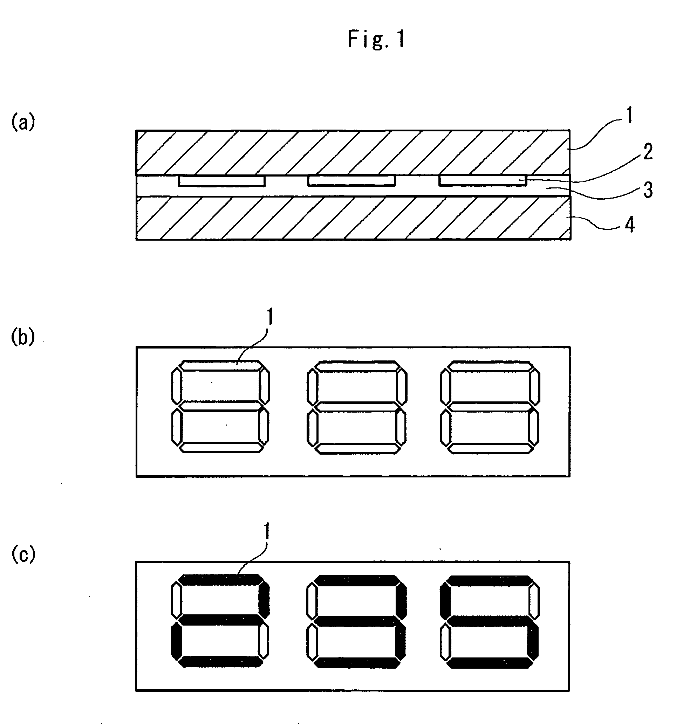

Fig. 1 shows views diagrammatically explaining the display device of the invention, wherein (a) is a cross-sectional view, (b) is a top view of the undisplayed state, and (c) is a top view of the displayed state. As shown in Fig. 1, the display device of the invention has, between a transparent substrate 1 having a surface electrode and a substrate 4 having a back electrode, a pattern display layer 2 containing an organic-inorganic hybrid polymer represented by the formula (I) or (II) below and a polymer gel electrolyte-containing layer 3. The surface electrode and back electrode constitute a counter electrode.

The transparent substrate having a surface electrode has a transparent surface electrode, such as an ITO electrode, and, as the transparent substrate, for example, a transparent substrate which is coated with a transparent conductive film having a light transmittance of 80% or more (a film of ITO, SnO₂, In₂O₃, or the like), and which has a surface resistance of 3 to 600 Ω can be used.

A light transmittance of the transparent substrate can be measured in accordance with the method for measuring a total light transmittance described in JIS K7105. As the transparent substrate, for example, glass or a polymer film can be used.

The glass means a substrate which is transparent with respect to a visible light or the like, and there can be used general glass mainly made of silicon dioxide, glass made of an inorganic material having various compositions, or resin glass using an organic material, such as a transparent acrylic resin or polycarbonate resin.

Examples of polymer films include polyester films, such as polyethylene terephthalate, polyolefin films, such as polypropylene, and resin films, such as polyvinyl chloride, an acrylic resin film, a polyether sulfone film, a polyallylate film, and a polycarbonate film, but preferred is a polyethylene terephthalate film because it has excellent transparency and is excellent in shapability, adhesion, processability, and the like.

The surface electrode of the transparent substrate preferably has a thickness of 10 to 5,000 nm, and, with respect to the thickness of the transparent substrate, there is no particular limitation. For example, when the transparent substrate is glass, it preferably has a thickness of 1 to 15 mm, and when the transparent substrate is a polymer film, it preferably has a thickness of 10 to 200 µm.

For preventing a short-circuiting phenomenon caused due to a narrow gap between the substrates and foreign matter or the like incorporated to the gap, a substrate having a transparent insulating layer having a thickness of about 200 to 1,000 Å formed on the transparent conductive film constituting the surface electrode may be used.

The substrate having a back electrode may be either the above-mentioned transparent substrate or an opaque substrate. For example, a stainless steel plate can be used as a substrate in the counter electrode, enabling production of an electronic paper unlikely to suffer breakage.

Alternatively, as the back electrode, a conductive metal thin film of aluminum, gold, silver, or the like may be used.

The display device of the invention can be produced as follows.

A coating composition of the organic-inorganic hybrid polymer is first prepared, and applied to a transparent substrate having a surface electrode and dried. The coating composition of the organic-inorganic hybrid polymer can be prepared by dissolving the organic-inorganic hybrid polymer in a volatile solvent capable of dissolving the organic-inorganic hybrid polymer, such as methanol.

After drying, a desired pattern is formed. For example, a pattern can be easily formed by the methods shown in items (i) to (iii) below.
(i) Method in which a polymer dissolved in methanol is applied by spin coating and then an unnecessary portion of the applied polymer is wiped away using methanol
An example of the method is as follows. An organic-inorganic hybrid polymer, such as Fe-MEPE, is dissolved in methanol to prepare a solution having a concentration of 20 mg/mL (2.8 mM)(the concentration is arbitrary, and the polymer is dissolved at, for example, a concentration in the range of from 1 to 100 mg/mL). The organic-inorganic hybrid polymer dissolved in methanol is applied to an electrode substrate, such as an ITO, by spin coating (for example, at 300 rpm for 200 sec). The number of revolutions for spin coating and the spin coating time are arbitrary, and a film having a desired thickness (color density) can be obtained by controlling the number of revolutions and the time of revolution.

An unnecessary portion of the polymer film is wiped away with a cloth, a rolling pin, or the like soaked with methanol, thus forming a desired pattern.
(ii) Method in which application is performed while heating using a hot plate
An example of the method is as follows. An organic-inorganic hybrid polymer, such as Fe-MEPE, is dissolved in methanol to prepare a solution having a concentration of 20 mg/mL (2.8 mM)(the concentration is arbitrary, and the polymer is dissolved at, for example, a concentration in the range of from 1 to 100 mg/mL).

An electrode substrate, such as an ITO, is preliminarily heated using a hot plate (at 30 to 80°C), and a pattern is made from the polymer dissolved in methanol on the electrode substrate, such as an ITO, using a paint brush, a brush, or the like. Methanol as a solvent starts evaporating simultaneously with making a pattern, enabling formation of an arbitrary pattern.
(iii) Method in which a pattern is made using dip coating
An example of the method is as follows. An organic-inorganic hybrid polymer, such as Fe-MEPE, is dissolved in methanol to prepare a solution having a concentration of 20 mg/mL (2.8 mM)(the concentration is arbitrary, and the polymer is dissolved at, for example, a concentration in the range of from 1 to 100 mg/mL).

An electrode substrate, such as an ITO, is dipped into the above organic-inorganic hybrid polymer solution, and after a lapse of several minutes, the electrode is removed from the solution, thereby forming a film (dip coating). A film having a desired thickness (color density) is obtained by controlling the concentration of the polymer solution.

The formed film is then air-dried overnight to remove methanol inside the film. Then, an unnecessary portion of the polymer film is wiped away with a cloth, a rolling pin, or the like soaked with methanol, thus forming a desired pattern.

The pattern display layer can be formed on the surface of the transparent substrate having a surface electrode, on the surface of the substrate having a back electrode, or on both of these surfaces.

On the other hand, a coating composition of the polymer gel electrolyte is prepared, and applied to the transparent substrate having a surface electrode and/or the substrate having a back electrode so as to cover the pattern display layer of the organic-inorganic hybrid polymer.

The coating composition of the polymer gel electrolyte is applied using individually or a combination of a bar coater method, an applicator method, a doctor blade method, a roll coater method, a die coater method, a comma coater method, a gravure coating method, a roll brush method, a spray coating method, an air knife coating method, an impregnation method, a curtain coating method, and the like.

In the application of the coating composition, if necessary, the coating composition may be diluted with an appropriate solvent. When using a solvent, the coating composition applied to the substrate is required to be dried. As mentioned above, the coating film is optionally formed on either one of or both of the transparent substrate having a surface electrode and the substrate having a back electrode.

With respect to the solvent, one which dissolves the electrolyte material and which can be removed by drying or the like after each application may be used, and methyl ethyl ketone, acetone, tetrahydrofuran, toluene, heptane, cyclohexane, ethyl acetate, ethanol, methanol, 2-propanol, isoamyl acetate, hexyl acetate, N,N-dimethylformamide, N-methyl-2-pyrrolidone, water, or the like can be used.

The surface electrode of the transparent substrate and the back electrode of the substrate are individually connected to the power source, and a predetermined voltage is applied to the pattern display layer containing the organic-inorganic hybrid polymer represented by the formula (I) or (II) and the polymer gel electrolyte-containing layer, making it possible to control the oxidation-reduction of the pattern display layer.

When the pattern display layer includes the single organic-inorganic hybrid polymer represented by the formula (I), the color of the layer may be controlled to be turned on or off by oxidizing or reducing the metal ion in the pattern display layer.

When the pattern display layer includes the organic-inorganic hybrid polymer represented by the formula (II), the color of the layer can be controlled to be turned on or off by controlling the potential so that the metal ions of plural types are individually oxidized or reduced.

The organic-inorganic hybrid polymer exhibits a color based on the charge-transfer absorption from the metal to the organic portion {bis(terpyridine) derivative} in the polymer. Specifically, when the organic-inorganic hybrid polymer is electrochemically oxidized, the color of the polymer disappears, and when the polymer in the colorless state is electrochemically reduced, the state of the polymer is turned to the colored state again.

In the organic-inorganic hybrid polymer, electron transfer occurs between the bis(terpyridine) derivative as a ligand and the metal ion in the presence of the polymer gel electrolyte. Appropriate selection of a metal ion causes the polymer to exhibit a color of blue, red, or the like. The charge transfer speed varies depending on the combination of the bis(terpyridine) derivative as a ligand and the metal ion, and therefore a desired color of the polymer can be obtained by appropriately selecting a combination of the bis(terpyridine) derivative as a ligand and the metal ion. The charge transfer speed can also be controlled by changing the counter anion.

Specifically, when an iron ion is selected as the metal ion and an acetate ion is selected as the counter anion, a blue to violet color can be exhibited, and when the acetate ion is changed to polyoxometalate, the blue to violet color is changed to deep blue (indigo). Further, when a cobalt ion is selected as the metal ion and an acetate ion is selected as the counter anion, a reddish brown color can be exhibited, and when the acetate ion is changed to polyoxometalate, the reddish brown color can be changed to blue.

Further, the organic-inorganic hybrid polymer turns a color on and off repeatedly by controlling the potential. These phenomena are reversible by controlling the potential. For example, with respect to the metal ions M¹ to M^{N} of the formula (II), the charge transfer speeds between the respective metal ions and the bis(terpyridine) derivative as a ligand are different from each other, and therefore the respective metal ions can cause the polymer to exhibit different colors.

Accordingly, appropriate selection of a combination of metal ions for different colors enables the organic-inorganic hybrid polymer to exhibit plural colors.

Further, in the organic-inorganic hybrid polymer of the formula (II), the metal ions M¹ to M^{N} have different oxidation-reduction potentials, and therefore only a color based on a specific metal ion can be exhibited by controlling the potential.

In the invention, the pattern display layer in an arbitrary form for segment display or the like is formed from the organic-inorganic hybrid polymer, and therefore the potential driving enables switching of display/non-display for a pattern in the pattern display layer and further switching of the types of colors exhibited. Furthermore, display is able to last for a long time (for example, for 30 minutes or longer) after shutting the power source off (memory effect).

For example, a color electronic paper is produced using an ITO electrode arranged for digital display, and can be used as a clock or the like.

Further, a desired pattern is made by applying the organic-inorganic hybrid polymer while heating an ITO substrate using a hot plate, enabling production of a large-size color electronic paper having a side of 40 cm.

The display device of the invention is preferably used in, for example, a color electronic paper, a color electronic poster, and a color electronic signboard.

The display device of the invention can be directly used, but, according to the use of the display device, the display device of the invention may be used in a way such that, for example, the display device is sandwiched between two supports or attached to one surface of a support. As the support, there can be used glass, a polymer film, or the like, which is used as the above-mentioned transparent substrate.

### [Examples]

Hereinbelow, the present invention will be described in more detail with reference to the following Examples, which should not be construed as limiting the scope of the invention.

### <Example 1>

### [Synthesis of an organic-inorganic hybrid polymer]

In a 100-ml two-neck flask, 30 mg (0.0054 mol) of 1,4-bis(terpyridyl)benzene was dissolved in 2.5 ml of acetic acid while heating. Then, 5 ml of a methanol solution containing 9.39 mg (0.0054 mo1) of iron(II) acetate was added to the two-neck flask. The resultant mixture was heated under reflux in a nitrogen gas atmosphere at 150°C for 24 hours.

After heating under reflux, the reaction solution in the two-neck flask was subjected to filtration, and then transferred to a Petri dish and dried in air to obtain an organic-inorganic hybrid polymer (Fe-MEPE) in the form of violet powder. The yield of the powder was 90%.

### [Preparation of a gel electrolyte]

2.1 g of PMMA (polymethyl methacrylate) was dissolved in acetonitrile, and the resultant solution was stirred overnight to prepare a solution A.

LiClO₄ was dissolved in acetonitrile (0.9 g/6 mL), and further 6 mL of propylene carbonate was added thereto to prepare a solution B.

The solution A and solution B were mixed together to obtain a gel electrolyte.

### [Production of a display device]

A 20 mg/mL (2.8 mM) methanol solution of the organic-inorganic hybrid polymer was applied to a segment-deposited ITO glass (manufactured by GEOMATEC Co., Ltd.; 10 Ω/cm) by spin coating (300 rpm, 200 sec), and air-dried overnight.

After a film was formed, an unnecessary portion of the film was wiped away using methanol.

Then, the gel electrolyte was applied to the entire surface of the formed Fe-MEPE film, and air-dried overnight. Similarly, the gel electrolyte was applied to an ITO glass as a counter electrode. The two electrodes were joined together and the excess gel electrolyte pushed out of the electrodes was removed, thus producing a color electronic paper having a pattern display layer of the Fe-MEPE digital display (7 segments). As a driving system, an apparatus using a dry cell as a power source (maximum: 6 V) was used (about 1.5 V per segment).

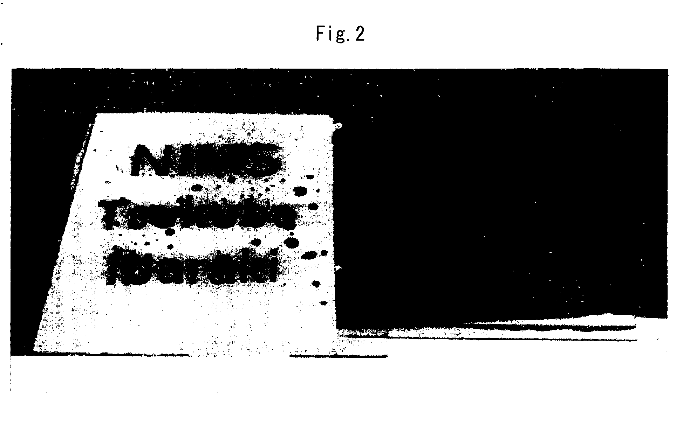

The produced color electronic paper realized smooth display of the figures from 0 to 9, and achieved segment display within one second as driving properties (right in Fig. 2). Further, an effect such that the display lasts for 30 minutes or longer after shutting the power source off (memory effect) has been confirmed.

### <Example 2>

### [Synthesis of an organic-inorganic hybrid polymer]

Fe-MEPE was synthesized in the same manner as in Example 1.

### [Preparation of a gel electrolyte]

2.1 g of PMMA (polymethyl methacrylate) was dissolved in 21 mL of acetonitrile, and the resultant solution was stirred overnight to prepare a solution A.

LiClO₄ was dissolved in acetonitrile (0.9 g/6 mL), and further 6 mL of propylene carbonate was added thereto to prepare a solution B.

50 mL of a mixture C of the solution A and solution B and 50 mg of titanium dioxide were mixed together to obtain a gel electrolyte.

### [Production of a display device]

A 20 mg/mL (2.8 mM) methanol solution of the organic-inorganic hybrid polymer was applied to a segment-deposited ITO glass (manufactured by GEOMATEC Co., Ltd.; 10 Ω/cm) by spin coating (300 rpm, 200 sec), and air-dried overnight.

After a film was formed, an unnecessary portion of the film was wiped away using methanol.

The gel electrolyte was applied to the entire surface of the formed Fe-MEPE film, and air-dried overnight. Similarly, the gel electrolyte was applied to an ITO glass as a counter electrode. The two electrodes were joined together and the excess gel electrolyte pushed out of the electrodes was removed, thus producing a color electronic paper having a pattern display layer of the Fe-MEPE digital display (7 segments). As a driving system, an apparatus using a dry cell as a power source (maximum: 3 V) was used.

The produced color electronic paper realized smooth display of the figures from 0 to 9, and achieved segment display within one second as driving properties (left in Fig. 2). A device exhibiting excellent electrochromic behavior even in the presence of titanium dioxide and having excellent visibility was successfully produced.

### <Example 3>

### [Synthesis of an organic-inorganic hybrid polymer]

Fe-MEPE was synthesized in the same manner as in Example 1.

### [Preparation of a gel electrolyte]

A gel electrolyte was prepared in the same manner as in Example 2.

### [Production of a display device]

An ITO glass (10 Ω/cm) and a stainless steel plate were used as substrates. A 20 mg/mL (2.8 mM) methanol solution of the organic-inorganic hybrid polymer was applied to a segment-deposited ITO glass (manufactured by GEOMATEC Co., Ltd.; 10 Ω/cm) by spin coating (300 rpm, 200 sec), and air-dried overnight.

After a film was formed, an unnecessary portion of the film was wiped away using methanol.

The gel electrolyte was applied to the entire surface of the formed Fe-MEPE film, and air-dried overnight. Similarly, the gel electrolyte was applied to a stainless steel plate as a counter electrode. The two electrodes were joined together and the excess gel electrolyte pushed out of the electrodes was removed, thus producing a color electronic paper having a pattern display layer of the Fe-MEPE digital display (7 segments). As a driving system, an apparatus using a dry cell as a power source (maximum: 3 V) was used.

The produced color electronic paper realized smooth display of the figures from 0 to 9, and achieved segment display within one second as driving properties. Even when a stainless steel plate was used as an electrode, excellent electrochromic behavior was exhibited.

### <Example 4>

### [Synthesis of an organic-inorganic hybrid polymer]

An organic-inorganic hybrid polymer (Ru-MEPE) was synthesized at a yield of 95% under substantially the same conditions as those in Example 1 except that, instead of iron acetate, ruthenium chloride•4 dimethyl sulfoxide {Ru(DMSO)₄Cl₂} was used, and that ethylene glycol was used as a solvent.

### [Preparation of a gel electrolyte]

A gel electrolyte was prepared in the same manner as in Example 1.

### [Production of a display device]

A color electronic paper having a large area was produced in substantially the same manner as in Example 1 except that Ru-MEPE and the Fe-MEPE in Example 1 were mixed in a 3:1 ratio and the resultant mixture was dissolved in methanol and a pattern was formed on ITO glass (10 inch size) x 8 (device body: made of polycarbonate) by the methods mentioned in the items (i) to (iii) above.

When voltages of 0 V, 0.8 V, and 2 V were individually applied to the produced device, the color of the device was changed, respectively, to reddish violet, orange, and yellowish green. Segment display within one second was achieved as driving properties, and multi-color display was achieved.

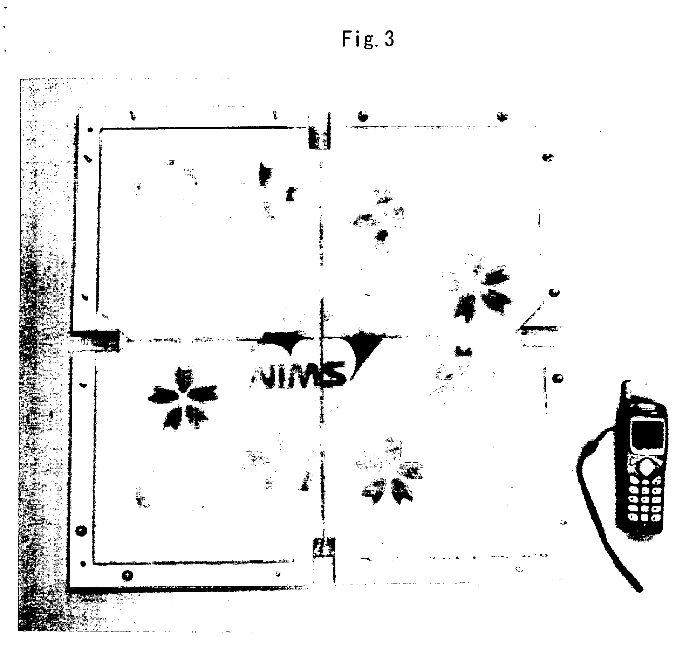

When the ratio of the two organic-inorganic hybrid polymers mixed, i.e., Ru-MEPE and Fe-MEPE is changed, different colors are exhibited at individual voltages. Further, with respect to the combination of the polymers mixed, not only in the case using a combination of Ru-MEPE and Fe-MEPE, but also in the case using Co-MEPE cobalt, Ni-MEPE, and the like, a similar multi-color change was confirmed. Furthermore, the pattern display layer was formed on not only the surface of the transparent substrate having a surface electrode but also the surface of the substrate having a back electrode to produce a color electronic paper, and driving properties and display properties similar to those mentioned above were obtained, thus achieving multi-color display with a large area (Fig. 3).

### <Example 5>

### [Synthesis of an organic-inorganic hybrid polymer]

1,4-Bis(terpyridyl)benzene, which is the same as that used in Example 1, and nickel(II) acetate tetrahydrate were mixed in a 1:1 molar ratio in ethanol, and heated in the solvent at 80°C for 18 hours while stirring. The obtained yellow solution was transferred to a Petri dish, and the solvent was distilled off to quantitatively obtain a desired product.

The obtained organic-inorganic hybrid polymer Ni-MEPE had an absorption at 483 nm in an ultraviolet-visible absorption spectrum, and an oxidation-reduction potential of the polymer was measured by differential pulse voltammetry (DPV), and, as a result, the oxidation-reduction potential was found to be 1.69 V.

### [Preparation of a gel electrolyte]

A gel electrolyte was prepared in the same manner as in Example 1.

### [Production of a display device]

A color electronic paper was produced in the same manner as in Example 1.

The produced color electronic paper realized smooth display of the figures from 0 to 9, and achieved segment display within one second as driving properties. Further, an effect such that the display lasts for 30 minutes or longer after shutting the power source off (memory effect) has been confirmed.

## Claims

1. A display device **characterized by** comprising, between a transparent substrate having a surface electrode and a substrate having a back electrode opposite to the surface electrode, a pattern display layer containing an organic-inorganic hybrid polymer represented by the following formula (I) or (II): 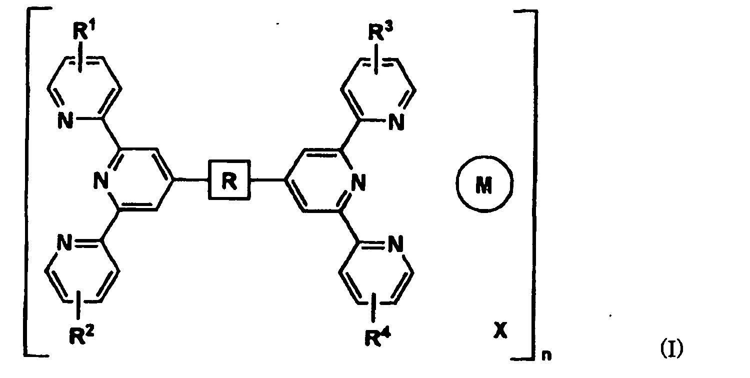 wherein M represents a metal ion, X represents a counter anion, R represents a spacer comprising a carbon atom and a hydrogen atom or a spacer for directly connecting two terpyridyl groups, each of R¹ to R⁴ independently represents a hydrogen atom or a substituent, and n represents an integer of 2 or more, which indicates a degree of polymerization, 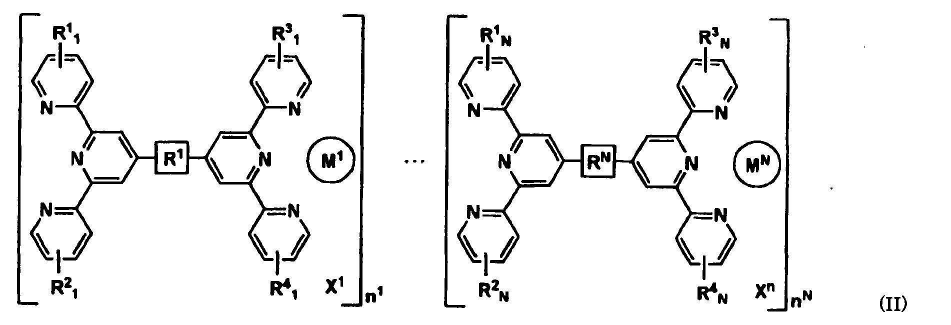 wherein each of M¹ to M^{N} (wherein N represents an integer of 2 or more) independently represents a metal ion, each of X¹ to X^{N} (wherein N represents an integer of 2 or more) independently represents a counter anion, each of R¹ to R^{N} (wherein N represents an integer of 2 or more) independently represents a spacer comprising a carbon atom and a hydrogen atom or a spacer for directly connecting two terpyridyl groups, each of R¹₁ to R¹_{N}, R²₁ to R^{?}_{N}, R³_{N} to R³_{N}, and R⁴₁ to R⁴_{N} (wherein N represents an integer of 2 or more) independently represents a hydrogen atom or a substituent, and each of n¹ to n^{N} (wherein N represents an integer of 2 or more) independently represents an integer of 2 or more, which indicates a degree of polymerization,
and a polymer gel electrolyte-containing layer.

2. The display device according to claim 1, **characterized in that** the pattern display layer is formed on the surface of the transparent substrate having a surface electrode and/or the substrate having a back electrode.

3. The display device according to claim 1 or 2, **characterized in that** the polymer gel electrolyte contains white particles.

4. The display device according to claim 3, **characterized in that** the white particles are titanium dioxide fine particles.

5. The display device according to any one of claims 1 to 4, **characterized in that** the metal ion in the organic-inorganic hybrid polymer is at least one member selected from an iron ion, a cobalt ion, a nickel ion, a zinc ion, and a ruthenium ion.

6. The display device according to any one of claims 1 to 5, **characterized in that** the counter anion in the organic-inorganic hybrid polymer is at least one member selected from an acetate ion, a chloride ion, a hexafluorophosphate ion, a tetrafluoroborate ion, and a polyoxometalate.

7. A color electronic paper **characterized by** comprising the display device according to any one of claims 1 to 6.

8. The color electronic paper according to claim 7, **characterized by** having a display portion having a size of 20 inches or more.
